# EUROPEAN PATENT SPECIFICATION

(11) **EP 0 426 486 B1**
(45) Date of publication and mention of the grant of the patent: **22.01.1997**
(21) Application number: 90312021.0
(22) Date of filing: 02.11.1990
(51) Int. Cl.: A61L 33/00, C08L 5/10

(54) **Anti-thromobogenic, and/or anti-microbial composition**
Antithrombogene und/oder antimikrobielle Zusammensetzungen
Compositions antithrombogéniques et/ou microbicides

(30) Priority: 02.11.1989 US 430340; 12.07.1990 US 551924
(43) Date of publication of application: 08.05.1991
(73) Proprietor: STS Biopolymers, Inc., Rush, New York 14523 (US)
(72) Inventor: Whitbourne, Richard James, Fairport, New York 14450 (US); Mangan, Margaret Anne, Rochester, New York 14620 (US)
(74) Representative: W.P. THOMPSON & CO.

(56) References cited:
- EP-A- 0 184 465
- EP-A- 0 231 573
- EP-A- 0 338 418
- WO-A-86/00795
- US-A- 3 695 921

## Description

The present invention relates to compositions containing heparin and/or antibiotic or other pharmaceutical agents. The compositions may have anti-thrombogenic and/or anti-microbial properties.

Many kinds of polymer compositions have been used in the field of medical supplies. These compositions have not always exhibited anti-thrombogenic characteristics when used in prosthetic and thereapeutic apparatuses for handling or being in contact with blood or blood components under conditions where clotting would tend to occur, such as artificial blood vessels, catheters, artificial hearts, and artificial kidneys.

When blood is brought in contact with metal, glass, plastic or other similar surfaces, it tends to clot in a short time unless certain precautions are taken. One common precaution currently in widespread use is the treatment of the surface with heparin or heparin reacted with quaternary ammonium compounds. Such heparin compounds are known to have anti-coagulant effects when in contact with blood. The presence of the aforementioned heparin compounds on the surface imparts anti-thrombogenic characteristics. However, previously known heparinization or compositions have not been adequate because of the short time of anti-thrombogenic activity, at most a few days in vivo (I. S. Hersch, et al, J. Biomed., Mater. Res. Symposium I, 99-104 (1971); K. Amplatz, "A Simple Non-Thrombogenic Coating", Invest. Radiology, July, August, 1971, Vol. 6) or because the anti-thrombogenic characteristic was reduced to a very low level in order to make it resistant to removal by reacting it with quaternary ammonium polymers (U.S. Patent 3,844,989).

EP-A-0184465 (Warner-Lambert Company) discloses an antithrombogenic thermoplastic polyurethane product, and process for preparing the same, the product comprising a substrate and at least one layer of a polyurethane alloy complex comprising a thermoplastic polyurethane and completely dispersed therein a preformed complex of an antithrombogenic material and/or an antibiotic agent ionically bonded with a quaternary ammonium compound.

EP-A-0338418 (Becton Dickinson and Company) discloses a leach resistant composition which includes a quaternary ammonium complex of heparin and silicone. A method for applying a coating of the composition to a surface of a medical article is also disclosed. Medical articles having surfaces which are both lubricious and antithrombogenic, are produced in accordance with the method.

It is therefore, an object of the present invention to provide anti-thrombogenic polymer/heparin compound compositions or mixtures which prevent blood clotting for a relatively long period of time (over one month), and which have the same high degree of anti-thrombogenic characteristics as the non-polymerized herapin-quaternary ammonium compounds, and thus provide excellent properties for use as medical materials for coatings on artificial blood vessels, catheters, artificial hearts, artificial kidneys, etc.

Another object of the present invention is to provide novel anti-microbial surfaces which contain antibiotic agents which are entrained in the surface in such a way as to be gradually released in vivo to provide effective anti-microbial action over a longer time than was previously possible when using these agents. Typical agents useful in this embodiment of the invention include penicillins, cephalosporins, etc.

### SUMMARY OF THE INVENTION

The present invention provides a composition comprising a mixture of a first component which comprises at least one of
(a) heparin reacted with a quaternary ammonium compound,
(b) an ionic antibiotic agent reacted with an ionic organic surfactant or ionic macromolecule, and
(c) an ionic pharmaceutical agent reacted with an ionic organic surfactant or ionic macromolecule,
and a second component comprising a water-insoluble cellulose ester polymer.

The invention also provides a method of forming a coating on the surface of a medical device to be brought into contact with human or animal body fluids, the method comprising
(a) forming a coating solution which comprises a composition according to the invention and a co-solvent for the first and second components of the composition,
(b) applying the coating solution to the surface of the device, and
(c) allowing the solution to dry.

Further, the invention provides a method of forming a coating on the surface of a medical device to be brought into contact with human or animal body fluids, the method comprising
(a) forming respective coating solutions comprising the first and the second components of a composition according to the invention and respective solvents,
(b) applying the coating solutions separately to the surface of the device, and
(c) allowing the solutions to dry.

The invention additionally provides a method of forming a coating on the surface of a water-insoluble polymer, the method comprising
(a) forming a coating solution which comprises the first component of a composition according to the invention and a solvent for the first component,
(b) applying the coating solution to a surface of a water-insoluble polymer comprising the second component of a composition according to the invention, and
(c) allowing the solution to dry.

Also provided is a medical device having at least a portion of its surface coated with the first component of a composition according to the invention bound with the second component thereof.

Anti-thrombogenic, anti-microbial compositions (mixtures) of this invention may thus comprise heparin-quaternary ammonium compounds and/or other ionic pharmaceutical agent-ionic surfactant or ionic macromolecular compounds mixed with water-insoluble cellulose ester polymers. They may also contain some hydrophilic polymers, but the mixture would still be water-insoluble after coating and drying. The water-insoluble polymers of compositions of this invention range from hydrophobic polymers to ones that are fairly hydrophilic, but are nevertheless essentially water-insoluble after being coated on a substrate and dried. A single polymer or mixture(s) of different polymers may be used to accomplish the invention. The heparin-quaternary ammonium compound may be mixed in a solution with the water-insoluble polymer, or it may be coated on top of a coating of the water-insoluble polymer(s), which is applied to the surface beforehand. In the latter case, a solvent must be added that is a mutual solvent for both the heparin-quaternary ammonium compound and the water-insoluble polymer(s) so that some mixing occurs between the two layers. In still another, case, it is possible to coat the heparin-quaternary ammonium compound directly on the water-insoluble plastic surface, and incorporate a mutual solvent for both the plastic surface and the heparin-quaternary ammonium compound, so that some mixing occurs between the plastic surface and the heparin-quaternary ammonium compound.

Various combinations of these three systems would be obvious to one skilled in the art. The mixtures of the water-insoluble polymer(s) and heparin-quaternary ammonium compounds of this invention are substantially more resistant to removal or deactivation in human and animal body fluids such as blood or plasma than the heparin-quaternary ammonium compounds by themselves.

Typical examples of polymers suitable for use with the present invention are as follows: Water insoluble cellulose esters such as cellulose acetate, cellulose acetate butyrate, cellulose acetate propionate, and cellulose nitrate; polyurethane resins including polyether and polyester grades. Exemplary of the polyurethane which the water-insoluble polymer may further comprise is the reaction product of 2,4-tolylene diisocyanate and position isomers thereof, 4,4'-diphenylmethane diisocyanate and position isomers thereof, polymethylenepolyphenyl isocyanate, or 1,5-naphthalene diisocyanate with 1,2-polypropylene glycol, polytetramethylene ether glycol, 1,4-butanediol, 1,4-butylene glycol, 1,3-butylene glycol, poly(1,4-oxybutylene)glycol, caprolactone, adipic acid esters, phthalic anhydride, ethylene glycol, 1,3-butylene glycol, 1,4-butylene glycol or diethylene glycol. Examples of acrylic polymers which the water-insoluble polymer may further comprise are ethyl and methyl acrylate and methacrylate; examples of condensation polymers which the water-insoluble polymer may further comprise are those produced by sulfonamides such as toluenesulfonamide and aldehydes such as formaldehyde; the water-insoluble polymer may also further comprise a polyisocyanate. Exemplary of the polyisocyanate are polymethylenepolyphenyl isocyanate, 4,4'-diphenylmethane diisocyanate and position isomers thereof, 2,4-tolylene diisocyanate and position isomers thereof, 3,4-dichlorophenyl diisocyanate and isoferrone isocyanate. Adducts or prepolymers of isocyanates and polyols such as the adduct of trimethylolpropane and diphenylmethane diisocyanate or tolylene diisocyanate are suitable. For further examples of polyisocyanates, see "Encyclopedia of Polymer Science and Technology", H. F. Mark, N. G. Gaylord and N. M. Bikales (eds.) (1969).

Typical quaternary ammonium compounds that can be reacted with heparin for use in this invention include benzalkonium chloride, tridodecylmethylammonium chloride, cetylpyrrdinium chloride, benzyldimethylstearylammonium chloride, benzylcetyldimethylammonium chloride, etc.

### DETAILED DESCRIPTION OF THE INVENTION

The compositions of the present invention may be first dissolved in solvent mixtures that are co-solvents for the mixtures of non-volatile components and which allow compatible homogenous films of the components to be cast. Such films when dried will typically appear as a clear film or films of very slight turbidity indicating that the non-volatile components have been deposited in a substantially homogenous manner. Typical solvents comprise alcohols, ketones, esters, aromatics, pyrrollidones, carboxylic acids, amides, and other organic solvents used alone or in appropriate mixtures as required, and which bring about the basic compatibility of the non-volatile components to be expressed. Typical surfaces which can be coated include plastic, metal and glass.

The heparin-quaternary ammonium compounds may be prepared in the conventional manner by any known prior art technique. For example, a heparin-benzalkonium chloride compound can be prepared by mixing approximately equal volumes of a 10% (by wt.) aqueous solution of sodium heparin with an approximately 17% (by wt.) solution of benzalkonium chloride (i.e., Zephiran® from Winthrop-Breon Laboratories), and then washing the residual sodium chloride out with distilled or deionized water. Such preparations are disclosed in "A Simple Non-Thrombogenic Coating", K. Amplatz, Invest., Radiology, July, August, 1971, Vol. 6. It should be understood, however, that the invention is not limited to the heparin-quaternary ammonium compounds cited in the above reference.

In most cases, all the components are incorporated into a single solution so that the surface treatment can be accomplished with a single application. However, the treatment can also be applied in two steps. For example, the water-insoluble cellulose ester polymer(s) can be applied in one application and the heparin-quaternary ammonium compound can be applied to the water-insoluble polymer. Some mutual solvent(s) for the water-insoluble polymer and heparin-quaternary ammonium compound that makes two components compatible should be included in the overcoat application to accomplish the objective of the invention. For example, dimethylacetamide (DMA) effectively accomplishes this objective as shown in Example 1. A variant on this approach would involve application of the water-insoluble polymer(s) followed by application of a solution containing some water-insoluble polymers and some heparin quaternary ammonium compound. Some heparin-quaternary ammonium compounds may also be added to the first application. Typical concentrations of heparin-quaternary ammonium compound in the coating solutions range from 0.1% to 20% by weight. Preferred concentrations range from 0.5% up to 4%. Use of higher concentrations of heparin-quaternary ammonium compounds in the solutions does not enhance performance and is therefore not very useful or desired. Lower concentrations than those disclosed above reduce the anti-thrombogenicity of the layers.

Typical concentrations of the water-insoluble polymers in the coating solution range from .01% to 20% by weight. Preferred concentrations range from .2% to 3%. Higher concentrations tend to mask the anti-thrombogenic characteristics of the layers. Lower concentrations tend to allow the layer to be extracted more easily. The composition of the final coating may have the heparin quaternary compound present in a concentration of 0.5 to 99.5 percent by weight with the balance of the composition comprising essentially the water-insoluble polymer.

### ANTI-THROMBOGENICITY TEST

The following in vitro test was used to evaluate anti-thrombogenicity: 10mm x 75mm glass test tubes were charged with 0.5 gm of reconstituted human plasma which had been kept refrigerated since collection. The test tubes were equilibrated in a 37°C incubator for 10-30 minutes. Next, 0.1 g of 0.10 M CaCl₂ was added, and the test tube was manually swirled to achieve complete mixing. Immediately after swirling, 4-1/2" (114 mm), long sections of 7 French tubing (either coated with one of the anti-thrombogenic systems of the present invention, or uncoated controls) were dropped into the plasma in each tube, taking care to ensure that the sample pieces were completely immersed in the plasma. The tubes were maintained in the 37°C incubator and were checked for clotting at one minute intervals by removing them from the incubator and tilting them. Before clotting, the liquid flows in the test tube, but it gels and does not flow once it has clotted. Typical clotting times for plasma containing untreated polyurethane tubing range from six minutes to 15 minutes. Samples made according to this invention prevent clotting in this test. It was found that if the plasma did not clot after standing overnight, it would usually not clot for up to four weeks. Therefore, tests were usually discontinued if they had not clotted after standing overnight. Typical samples prepared by this invention did not clot when tested before plasma extraction, and retained their anti-clotting activity after 28 or more days of extraction in plasma. Devices coated with heparin-benzalkonium chloride or heparin-tridodecylmethylammonium chloride do not clot when tested before extraction in plasma, but lose their anti-thrombogenicity after plasma extraction of two hours or less. Heparinized quaternary polymers (HQP), such as those prepared according to U.S. Patent 3,844,989 and used on catheters marked under the trademark ANTHRON by Toray Medical Co. Ltd., show only slight anti-thrombogenicity. For example, when tested against heparin-benzalkonium chloride (HBAC), the HBAC sample prevented clotting of the plasma overnight, while the control clotted in five minutes and the HQP sample clotted in seven minutes before plasma extraction, and showed no improvement in anti-thrombogenicity compared to the untreated polyurethane control after 12 hours of plasma extraction.

In the following examples, Examples 1 to 9, 15 and 18 are in accordance with the present invention. The remaining examples are for comparison or reference purposes

### Example 1

Polyurethane 7 French tubing was coated with a solution containing the following ingredients and dried for 20 minutes at 65°C.

| | |
|---|---|
| Polyvinylpyrrolidone | .006 g |
| Isopropanol | 1.0 g |
| Nitrocellulose | 1.6 g |
| Ethylacetate | 1.2 g |
| Rosin ester | .5 g |
| Butylacetate | 4.8 g |
| Dimethylacetamide | 1.5 g |
| Ethyl-3-ethoxy propionate | 6.1 g |

The tubing was then overcoated with a solution containing the following ingredients and then dried for 20 minutes at 65°C.

| | |
|---|---|
| Isopropanol | 9.85 g |
| Dimethylacetamide | 1.00 g |
| Heparin benzalkonium chloride | .15 g |

This sample was compared to a sample of polyurethane tubing which was coated with heparin benzalkonium chloride (1.8% w/v in isopropanol) as follows. The samples were dipped in a Gentian Violet dye solution and then rinsed in hot running water. The sample coated with heparin-benzalkonium chloride (HBAC) in isopropanol lost most of the surface dye stain in less than 20 seconds, indicating that most of the HBAC had been washed off. The sample of the present invention that had the nitrocellulose undercoat and contained DMA in the HBAC overcoat, retained the dye stain much longer indicating that it is much more resistant to removal.

### Example 2

Polyurethane 7 French tubing was coated with a solution consisting of:

| | |
|---|---|
| Methylethylketone | 5.0 g |
| Heparin-benzalkonium chloride | 0.33 g |
| Isopropanol | 3.7 g |
| Ethyl-3-ethoxy propionate | .6 g |
| Butyl acetate | .5 g |
| 1/2 sec. nitrocellulose | .16 g |
| Ethyl acetate | .1 g |
| Rosin ester | .05 g |

The samples were dried at 75°C for 30 minutes. Samples were then extracted in human plasma at 37°C for 7, 10, 21, or 28 days and then tested for anti-clotting properties. The following results were obtained.

| Sample | Clotting time |
|---|---|
| Uncoated control | 12 minutes |
| Above sample, without extraction in plasma | Did Not Clot |
| Above sample, after 7 days extraction in plasma | Did Not Clot |
| Above sample, after 10 days extraction in plasma | Did Not Clot |
| Above sample, after 21 days extraction in plasma | 24 minutes |
| Above sample, after 28 days extraction in plasma | 20 minutes |

The above results show that the samples are still exhibiting effective anti-clotting activity on the device surface where it is most needed and that clots are unlikely to form on the treated surfaces, even after 28 days of extraction. This level of anti-clotting activity is stronger even after 28 days of plasma extraction than the anti-clotting levels achieved under these test conditions with surfaces treated according to the compositions taught by U.S. Patent 3,844,989.

### Example 3

The following solution was coated on polyurethane 7 French tubing and dried at 75°C for 20 minutes.

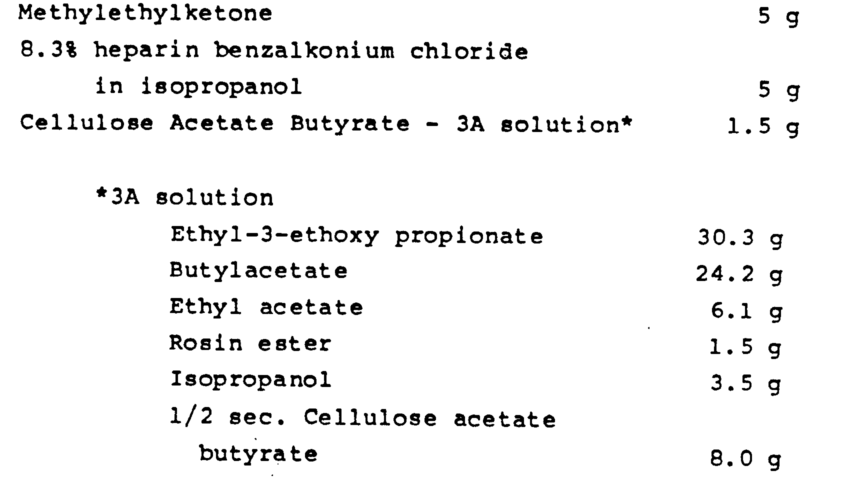

Coated samples were tested for anti-clotting activity, and also for resistance to removal by dying with Gentian Violet dye and then rinsing with hot running water. The sample was compared to a coating of heparin benzalkonium chloride without any cellulose ester polymer additive.

Results: The sample did not clot in the clotting test. In the hot water rinse test, the heparin benzalkonium chloride coating without cellulose resin was completely removed in a few seconds. Hot water rinsing did not remove the above coating which contained cellulose acetate butyrate polymer.

### Example 4

Polyurethane 7 French tubing was coated as in Example 3 except that cellulose acetate butyrate was replaced with cellulose acetate propionate. The sample was tested for anti-clotting activity and resistance to removal in hot water. Results were comparable to those with Example 3.

### Example 5

Polyurethane 7 French tubing was coated with the following solution and dried at 80°C for 20 minutes.

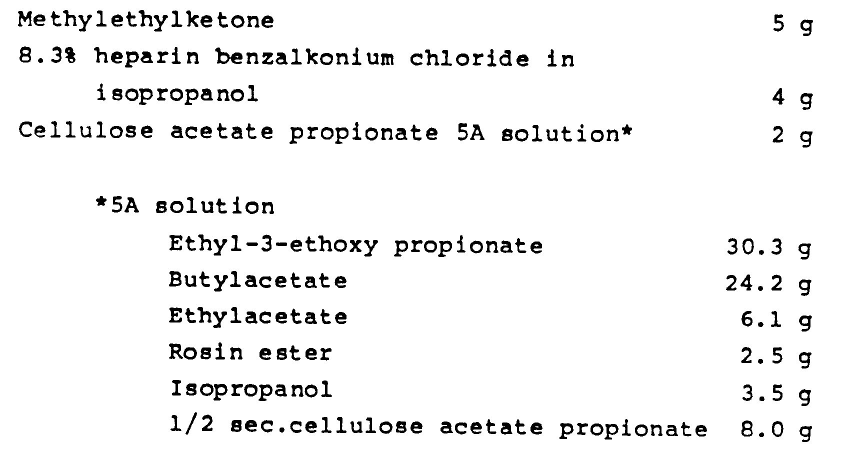

The coated sample was extracted in plasma at 37°C for four hours and tested for anti-microbial activity by, pressing it into gel led Difco Plate Agar which was spiked with Staphylococcus epidermidis (ATCC 12228) and then incubated overnight at 32-35°C. A sample of polyurethane tubing that was coated with heparin-benzalkonium chloride without cellulose polymer was extracted in plasma at 37°C for four hours for comparison. The sample which contained cellulose acetate propionate (CAP) polymer showed a significant zone of inhibition while the sample made without CAP resin showed no zone of inhibition, demonstrating that the incorporation of cellulose ester polymer effectively increases resistance to removal of the coating when extracted in human plasma.

### Example 6

Example 5 was repeated, except that the solution contained 1.5 gm of 10.7% (wt. %) nitrocellulose solution in place of the 2.0 grams of 10.7% (wt. %) CAP solution. Samples of polyurethane tubing coated with this solution were extracted in plasma at 37°C for four hours or 18 hours. They were then tested for anti-microbial activity using the same zone of inhibition test as used in Example 5. The tests showed zones of inhibition after both extraction intervals. The sample extracted for four hours has a larger zone of inhibition than the sample that was extracted for 18 hours.

### Example 7

The following solution was coated on polyurethane 7 French tubing and dried at 80°C for 20 minutes. A control was made by coating a sample of the tubing with a 5% w/v solution of Tridodecylmethylammonium chloride (TDMAC).

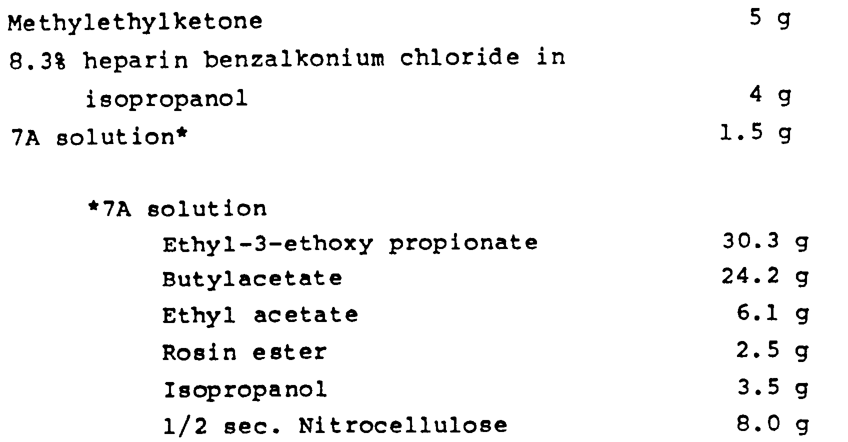

Both samples were then immersed for 30 minutes in a 5% aqueous solution of penicillin G and then air dried overnight. The coated samples were then extracted for 18 hours in human plasma at 37°C. They were removed from the plasma, rinsed in running deionized water and then tested for anti-microbial activity as in Example 5. The sample containing nitrocellulose showed a strong zone of inhibition while the sample without nitrocellulose showed no zone of inhibition.

### Example 8

Example 7 was repeated, except that TDMAC was added to the coating solutions as follows:

| | |
|---|---|
| Example 8 | .025 gm TDMAC added |
| Example 8A | .075 gm TDMAC added |

Both samples showed a strong zone of inhibition after the 18 hours plasma extraction and appeared to be substantially comparable to Example 7.

### Example 9

Polyurethane 7 French tubing was coated with the following solution.

| | |
|---|---|
| Heparin tridodecylmethylammonium chloride | 0.2 g |
| Isopropanol | 2.6 g |
| Methylethylketone | 2.5 g |
| 7A Solution | 0.7 g |

This coated sample was tested for clotting and did not clot. It was very resistant to removal in hot running water.

### Example 10 (Reference)

Polyurethane 7 French tubing was coated with a solution containing the following ingredients and dried at ambient temperature for 60 minutes:

| | |
|---|---|
| Methylethylketone | 5.3 g |
| Heparin-benzalkonium chloride | 0.31 g |
| Isopropanol | 3.4 g |
| Acrylic resin | 0.2 g |
| Rosin ester | 0.2 g |
| Tridodecylmethylammonium chloride | 0.4 g |
| Xylene | 0.14 g |
| Butanol | 0.05 g |

Samples were then extracted in plasma at 37°C for 4, 24 and 120 hours and compared to uncoated polyurethane tubing for anti-clotting activity. The results were as follows:

| Sample | Clotting Time |
|---|---|
| Uncoated control | 9 minutes |
| Above sample, without extraction in plasma | Did Not Clot |
| Above sample, after 4 hours extraction in plasma | Did Not Clot |
| Above sample, after 24 hours extraction in plasma | Did Not Clot |
| Above sample, after 120 hours extraction in plasma | Did Not Clot |

The above coated sample was resistant to removal by hot running water.

### Example 11 (Reference)

Polyurethane 7 French tubing was coated with a solution containing the following ingredients and dried 15 minutes at 75°C:

| | |
|---|---|
| Methylethylketone | 5.6 g |
| Heparin-benzalkonium chloride | 0.33 g |
| Isopropanol | 3.5 g |
| Polyurethane resin | 0.24 g |
| Polyisocyanate resin | 0.19 g |
| Ethyl acetate | 0.19 g |

Samples were extracted in plasma at 37°C for 72 hours and then tested for anti-clotting properties. A sample of polyurethane tubing which was coated with heparin-benzalkonium chloride (1.8% w/v in isopropanol) was also extracted in plasma at 37°C for 72 hours for comparison. The following results were obtained:

| Sample | Clotting Time |
|---|---|
| Uncoated control | 13 minutes |
| Above sample, after 72 hours extraction in plasma | Did Not Clot |
| Sample coated with heparin-benzalkonium chloride in isopropanol, after 72 hours extraction in plasma | 7 minutes |

The above coating was also resistant to removal by hot running water.

### Example 12 (Reference)

Polyurethane 7 French tubing was coated with a solution containing the following ingredients and dried for 20 minutes at 70°C.

| | |
|---|---|
| Methylethylketone | 5.9 g |
| Heparin-benzalkonium chloride | 0.32 g |
| Isopropanol | 3.5 g |
| Polyurethane resin | 0.14 g |
| Polyisoyanate resin | 0.07 g |
| Ethylacetate | 0.07 g |

Samples were then extracted in human plasma at 37°C for 3, 24, and 48 hours and then tested for anti-clotting properties. The following results were obtained:

| Sample | Clotting Time |
|---|---|
| Uncoated control | 8 minutes |
| Above sample, after 3 hours extraction in plasma | Did Not Clot |
| Above sample, after 24 hours extraction in plasma | Did Not Clot |
| Above sample, after 48 hours extraction in plasma | 9 minutes |

### Example 13 (Reference)

Polyurethane 7 French tubing was coated with a solution containing the following ingredients and dried for 20 minutes at 70°C.

| | |
|---|---|
| Methylethylketone | 6.1 g |
| Heparin-benzalkonium chloride | 0.32 g |
| Isopropanol | 3.5 g |
| Polyurethane resin | 0.07 g |
| Polyisoyanate resin | 0.04 g |
| Ethylacetate | 0.04 g |

Coated tubing was then extracted in plasma for 3 and 24 hours and then tested for anti-clotting behavior. The following results were obtained:

| Sample | Clotting Time |
|---|---|
| Uncoated control | 8 minutes |
| Above sample, after 3 hours extraction in plasma | Did Not Clot |
| Above sample, after 24 hours extraction in plasma | 9 minutes |

### Example 14 (Reference)

Polyurethane 7 French tubing was coated with a solution containing the following ingredients and dried for 20 hours at 55°C.

| | |
|---|---|
| Heparin tridodecylmethylammonium chloride | 0.32 g |
| Dimethylacetamide | 6.2 g |
| Toluene | 2.0 g |
| Petroleum ether | 1.5 g |

The coated tubing was extracted in human plasma at 37°C for 1, 2, 3 and 6 days and then tested for anti-clotting properties.

| Sample | Clotting Time |
|---|---|
| Uncoated sample | 10 minutes |
| Above sample, after 1 day extraction in plasma | Did Not Clot |
| Above sample, after 2 days extraction in plasma | Did Not Clot |
| Above sample, after 3 days extraction in plasma | Did Not Clot |
| Above sample, after 6 days extraction in plasma | Did not Clot |

The preceding examples, together with controls, show clearly that heparin-quaternary ammonium compounds that are not polymeric can be made more resistant to removal or deactivation in various body fluids such as whole blood or plasma (including human) by mixing with appropriate water-insoluble polymers. Coatings made from normal heparin-quaternary ammonium compounds by themselves using solvents that do not cause mixing with the substrate, such as heparin-benzalkonium chloride, or heparin tridodecylmethylammonium chloride show little anti-thrombogenicity after soaking in human plasma for only a few hours. The heparin-TDMAC compound continues to show anti-thrombogenicity somewhat longer than the benzalkonium chloride compound, but both exhibit almost no anti-thrombogenicity after soaking in human plasma for a few hours. The incorporation of water-insoluble polymers according to the present invention, and as shown in the examples, greatly extends the time for which coating samples can be soaked in human plasma and still show substantially levels of anti-thrombogenicity. For instance, some samples were found to show antithrombogenicity even after soaking in human plasma for 28 days.

On the other hand, when quarternary ammonium polymers are reacted with heparin, the coating remains on the surface even after long periods of soaking in body fluids such as human plasma, but the anti-thrombogenicity is not as strong either before soaking or after soaking for up to 28 days in human plasma, as in the samples made according to this invention. It is further noted that by water-insoluble polymers we are implying that they are water-insoluble after a film is cast and dried, and include water-insoluble polymers that may be hydrophilic, but nevertheless cause the heparin-quaternary ammonium compounds to remain anti-thrombogenic after prolonged soaking in body fluids.

In a further embodiment of the present invention, it has been found that it is possible to react an antibiotic or other pharmaceutical agent such as penicillin, ticarcillin, cefotoxin, cephalosporins, oxacillin, and carbonicillin that contains a positive inorganic ion such as sodium with a quaternary ammonium compound such as benzalkonium chloride or TDMAC to produce an ionic antibiotic that is soluble in organic solvents and is miscible with hydrophobic water insoluble polymers. In this embodiment, the resulting polymer mixture would not contain an anti-thrombogenic agent such as heparin. It is also possible to react other antibiotics or pharmaceutical agents that contain a negative ion such as chloride with surfactants including macromolecules that contain a negative organic ion such as sodium laurylsulfate or sodium stearate, or polyacrylic acid to again convert a water soluble antimicrobial agent or other pharmaceutical agent into one that is soluble in organic solvents and miscible with hydrophobic water insoluble polymers. When these organic solvent soluble agents are mixed with polymers of this invention, they can be rendered much more resistant to removal in plasma from the surface of an article coated with them than if they are coated on the surface without the polymer.

By making the antibiotic or other pharmaceutical agent soluble in organic solvents and miscible with the water insoluble polymers of this invention, it makes it possible to incorporate useful pharmaceuticals such as antibiotics onto medical devices at the time of manufacture. The pharmaceuticals are available at the surface of the device in efficacious concentrations, over a useful period such as several days to weeks. At the same time, while the pharmaceuticals are present in useful concentrations where they are wanted on the device surface, they are not present in high concentration systematically so that typical side effects normally associated with various pharmaceuticals are unlikely.

The polymer can be mixed with the pharmaceutical agent and then coated, or the polymer or agent can be coated first and then overcoated with the other agent. The single coating of a mixture of polymer and pharmaceutical agent would normally be preferred because it would involve only a single coating and because the ratio of pharmaceutical agent to polymer can be controlled more precisely. Such mixtures of organic ionic pharmaceutical agents and polymers would not be anti-thrombogenic unless they also contained an anti-thrombogenic agent such as heparin. However, the coatings do show strongly the desired effect of the incorporated pharmaceutical agent such as anti-microbial activity. The presence of certain polymers also has the added benefit of enhancing the stability of the pharmaceutical agent to a sterilization process such as exposure to ethylene oxide.

The antibiotic-surfactant compound is present in a concentration of 0.5% to 99.5% by weight with the balance comprising the water-insoluble polymer. The concentration of the water-insoluble polymer is typically 0.01% to 40% by weight and the concentration of antimicrobial-surfactant compound is 0.01% to 40% by weight of the coating solution.

The following example demonstrates how the system works.

In Examples 15-18, ¹⁴C-penicillin G sodium salt was reacted with tridodecylmethylammonium chloride (TDMAC) using procedures similar to those previously described in the Background Of The Invention, see A. Amplatz, "A Simple Non-Thrombogenic Coating", Invest. Radiology, July, August, 1971, Vol. 6.

A typical method of preparation for the pharmaceutical agent-TDMAC compounds of the present invention is as follows:

Seventeen grams TDMAC is dissolved in 60 ml isopropanol, and diluted with 40 ml distilled water. Next, dissolve 10 grams of the sodium salt of the pharmaceutical agent (SPA) in 100 ml distilled water. Mix equal volumes of both liquids and shake vigorously for ten or so seconds to ensure complete mixing and reaction.

Next, vacuum filter over filter paper, collect the compound off the paper, and place in a centrifuge jar with 1 volume of water, shake for 30 minutes, and vacuum filter again on filter paper. The wash is repeated twice more. The SPA-TDMAC is dried in an oven at 60°C.

Using this basic procedure, it is obvious to one skilled in the art that organic salts can be made from many or most ionic pharmaceutical agents by mixing them together with an appropriate ionic surfactant, or ionic macromolecule and washing out the water-soluble salt residue with water. These compounds are soluble in organic solvents and typically have very low solubility constants so that when mixed with the polymers of this invention, constant and efficacious concentrations of the pharmaceutical agent(s) will be available on the coated surface in vivo over an extended period.

The resultant ¹⁴C-penicillin-TDMAC prepared by the above method is soluble in various organic solvents and has extremely low water solubility but is still ionic. The ¹⁴C-penicillin-TDMAC was then mixed with selected polymers and coated on both silicone and polyurethane tubing. The coatings were then extracted in plasma for one day or 5 days and compared to non-extracted samples by scintillation counting to determine how much penicillin remained on the surface in the coating after extraction. Some samples were exposed to an ethylene oxide sterilization cycle and tested by zone of inhibition, to show whether the polymer improved the resistance of the antibiotic to degradation when exposed to ethylene oxide.

### Example 15

The ¹⁴C-penicillin-TDMAC was mixed with cellulose nitrate dissolved in a solvent mixture containing ethanol, isopropanol, ethylacetate and toluene. The solution has the following composition:

| | |
|---|---|
| Nitrocellulose | 1.8 g |
| Isopropanol | .8 g |
| Toluene | 24.3 g |
| Ethyl acetate | 5.1 g |
| Camphor | .5 g |
| Dibutylphthalate | .7 g |
| ¹⁴C-penicillin-TDMAC | 2.0 g |

The solution was coated on both silicone and polyurethane tubing and dried. Some coated samples were then extracted in plasma for 24 hours or five days. After plasma extraction, the samples were measured by scintillation counting and were compared to unextracted samples to show how much ¹⁴C-penicillin-TDMAC remained. The following results were obtained.

| | Not Extracted | After 24 h. Extraction | After Five Days Extraction |
|---|---|---|---|
| Silicone tubing | 48 µg/cm² | 30 µg/cm² | 6 µg/cm² |
| Polyurethane tubing | 36 µg/cm² | 43 µg/cm² | 38 µg/cm² |

When the ¹⁴C-penicillin-TDMAC was coated without polymer, it was removed from the tubing surface in a few hours. These results clearly show how incorporation of this polymer into the coating dramatically extends the elution time from the surface when extract in plasma.

### Example 16 (Reference)

Example 15 was repeated using Silastic® silicone resin in 1,1,1,-Trichlorethane in place of the nitrocellulose solution. The solution has the following composition:

| | |
|---|---|
| Silastic® Polymer | 1.3 g |
| 1,1,1-Trichloroethane | 28.7 g |
| Toluene | 8.0 g |
| ¹⁴C-penicillin-TDMAC | 2.0 g |

This sample was tested for resistance to extraction in plasma, and for resistance to degradation by ethylene oxide sterilization.

### Results:

| | Not Extracted | After 24 h. Extraction | After Five Days Extraction |
|---|---|---|---|
| Silicone tubing | 68µg/cm² | 33µg/cm² | 27µg/cm² |
| Polyurethane tubing | 18µg/cm² | 8µg/cm² | 6µg/cm² |

These results show that incorporation of Silastic® resin into the coating extends the elution time of the antibiotic in plasma to several days compared to a few hours without the resins.

After exposure to a typical ethylene oxide sterilization (ETO) cycle, the samples were tested by classic zone of inhibition testing. This was done by placing a sample (sterilized or non-sterilized) onto a layer of agar containing bacteria and then incubated. The results are reported as the size in mm of the clear zone surrounding the coated article which results from the antimicrobial activity of active ¹⁴C-penicillin-TDMAC.

| | Before ETO Exposure | After ETO Exposure |
|---|---|---|
| With Silastic® | 11 | 10 |
| Without polymer | 26 | 0 |

This result clearly demonstrates how incorporation of Silastic® polymer into the ¹⁴C-penicillin-TDMAC coating greatly increases the resistance of the antibiotic to degradation caused by exposure to ethylene oxide.

### Example 17 (Reference)

Example 15 was repeated using polyvinylbutyral (PVB) polymer in toluene in place of the nitrocellulose solution. The solution has the following composition:

| | |
|---|---|
| Polyvinylbutryral | 1.5 g |
| Toluene | 31.5 g |
| ¹⁴C-penicillin-TDMAC | 2.0 g |

This was coated on silicone and polyurethane tubings, dried, and then tested for resistance to extraction in plasma, and resistance to degradation during an ethylene oxide sterilization by zone of inhibition. The following results were obtained.

| | Not Extracted | After 24 h. Extraction | After Five Days Extraction |
|---|---|---|---|
| Silicone tubing | 18µg/cm² | 18µg/cm² | 16µg/cm² |
| Polyurethane tubing | 12µg/cm² | 13µg/cm² | 10µg/cm² |

| | Before ETO Sterilization | After ETO Sterilization |
|---|---|---|
| With PVB Polymer | 25 mm | 15 mm |
| Without Polymer | 26 mm | 0 |

Clearly, PVB polymer provides significant stabilization to ¹⁴C-penicillin-TDMAC against degradation caused by exposure to ethylene oxide.

### Example 18

Example 1 was repeated using cellulose acetate butyrate polymer (CAB) in place of the nitrocellulose solution. The solution has the following composition:

| | |
|---|---|
| Cellulose Acetate Butyrate | 2.0 g |
| Ethyl acetate | 8.0 g |
| Toluene | 17.0 g |
| ¹⁴C-penicillin-TDMAC | 2.0 g |

This solution was coated on silicone and polyurethane tubings, dried, and tested for resistance to extraction from the surface in plasma. It was also tested for resistance to degradation from ethylene oxide exposure. The following results were obtained.

| | Not Extracted | After 24 h Extraction | After Five Days Extraction |
|---|---|---|---|
| Silicone tubing | 33 µg/cm² | 12 µg/cm² | 17 µg/cm² |
| Polyurethane tubing | 20 µg/cm² | 12 µg/cm² | 7 µg/cm² |

CAB polymer clearly increases the resistance of ¹⁴C-penicillin-TDMAC to extraction in penicillin.

| | Before ETO Sterilization | After ETO Sterilization |
|---|---|---|
| With CAB Polymer | 31 mm | 31 mm |
| Without Polymer | 26 mm | 0 |

This result shows how CAB polymer provides substantial stabilization to ¹⁴C-penicillin-TDMAC against ETO induced degradation.

The foregoing Examples show how incorporation of these and/or other water insoluble polymers clearly improves resistance of pharmaceutical salts of organic ions to extraction in plasma and against the degradation effects from exposure to sterilization with ethylene oxide. At the same time, however, the incorporation of polymers still leaves effective concentrations of antibiotic available at the coated surface as demonstrated by the zone of inhibition test results in Examples 16, 17 and 18.

It is expected that different polymers could be used together in a single solution/coating or in contiguous layers to further enhance performance and achieve specific results. We have also tested other polymers mixed with the organic ion salts of pharmaceuticals and found similarly useful improvement in resistance to extraction by plasma. These include polyisocyanates, acrylicpolymers, vinylacetate, and others.

## Claims

1. A composition comprising a mixture of a first component which comprises at least one of
(a) heparin reacted with a quaternary ammonium compound,
(b) an ionic antibiotic agent reacted with a ionic organic surfactant or ionic macromolecule, and
(c) an ionic pharmaceutical agent reacted with an ionic organic surfactant or ionic macromolecule,
and a second component comprising a water-insoluble cellulose ester polymer.

2. A composition according to claim 1, in which the water-insoluble polymer further comprises at least one of a polyurethane resin, an acrylic polymer, a condensation polymer, an aldehyde condensation polymer and a polyisocyanate.

3. A composition according to claim 1 or 2, in which the water-insoluble cellulose ester polymer is at least one of cellulose acetate, cellulose acetate butyrate, cellulose acetate propionate, cellulose nitrate, and mixtures thereof.

4. A composition according to any preceding claim, in which the first component comprises (a) and the composition has anti-thrombogenic properties, the quaternary ammonium compound being at least one of benzalkonium chloride, tridodecylmethylammonium chloride, cetylpyridinium chloride, benzyldimethylstearylammonium chloride and benzylcetyldimethylammonium chloride.

5. A composition according to claim 4, in which the quaternary ammonium compound is benzalkonium chloride.

6. A composition according to claim 4 or 5, in which (a) is present in a concentration of at least 0.5% by weight of the composition.

7. A composition according to claim 6, in which (a) is present in a concentration of 0.5% to 99.5% by weight of the composition with the balance being the water-insoluble polymer.

8. A composition according to any of claims 4 to 6, in which the first component also contains (b) in which the ionic organic surfactant comprises a quaternary ammonium compound.

9. A composition according to any of claims 1 to 3, in which the first component comprises (b) and the composition has anti-microbial properties, the antibiotic agent being negatively charged and comprising one or more of penicillin, ticarcillin, cefotoxin, a cephalorsporin, oxacillin and carbonicillin, the surfactant being a quaternary ammonium compound.

10. A composition according to claim 9, in which (b) is present in a concentration of 0.5% to 99.5% by weight of the composition with the balance being the water-insoluble polymer.

11. A composition according to any of claims 8 to 10 in which the quaternary ammonium compound is at least one of benzalkonium chloride, tridodecylmethylammonium chloride, cetylpyridinium chloride, benzyldimethylstearylammonium chloride and benzylcetyldimethylammonium chloride.

12. A composition according to any of claims 1 to 3, in which the first component comprises (b) and the composition has anti-microbial properties, the antibiotic agent being positively charged and the surfactant having a negatively charged organic ion.

13. A composition according to claim 10, in which the surfactant comprises sodium lauryl sulphate, or stearic acid or a salt thereof or polyacrylic acid or a copolymer thereof.

14. A composition according to any of claims 8 to 13, in which (b) and the water-insoluble polymer are each present in a concentration of 0.01% to 40% by weight of the composition.

15. A composition according to any preceding claim, in which the first and second components are in separate layers.

16. A composition comprising a mixture as recited in any of claims 1 to 14 and a mutual organic solvent, the concentrations of the first and the second components in the solvent being from 0.1 to 20% by weight and from 0.01 to 20% by weight, respectively.

17. A method of forming a coating on the surface of a medical device to be brought into contact with human or animal body fluids, the method comprising
(a) forming a coating solution which comprises a composition according to any of claims 1 to 14 and a co-solvent for the first and second components of the composition,
(b) applying the coating solution to the surface of the device, and
(c) allowing the solution to dry.

18. A method of forming a coating on the surface of a medical device to be brought into contact with human or animal body fluids, the method comprising
(a) forming respective coating solutions comprising the first and the second components of a composition according to any of claims 1 to 14 and respective solvents,
(b) applying the coating solutions separately to the surface of the device, and
(c) allowing the solutions to dry.

19. A method of forming a coating on the surface of a water-insoluble polymer, the method comprising
(a) forming a coating solution which comprises the first component of a composition according to any of claims 1 to 14 and a solvent for the first component,
(b) applying the coating solution to a surface of a water-insoluble polymer comprising the second component of a composition according to any of claims 1 to 14, and
(c) allowing the solution to dry.

20. A method according to any of claims 17 to 19, in which the concentration of the first component in its respective coating solution is 0.1% to 20% by weight of the solution.

21. A method according to claim 17 or 18, in which the concentration of the second component in its respective coating solution is 0.01% to 20% by weight of the solution.

22. A medical device having at least a portion of its surface coated with the first component of a composition according to any of claims 1 to 14 bound with the second component of a composition according to any of claims 1 to 14.

23. A medical device according to claim 22, in which the coated portion is at least one of glass, metal, and plastic.

24. A medical device according to claim 22 or 23, the device being a catheter or an artificial blood vessel, heart or kidney.

## Patentansprüche

1. Zusammensetzung, enthaltend ein Gemisch aus einer ersten Komponente, die mindestens einen der folgenden Bestandteile enthält:
(a) mit einer quaternären Ammoniumverbindung umgesetztes Heparin,
(b) ein mit einem ionischen organischen Tensid oder einem ionischen Makromolekül umgesetztes ionisches antibiotisches Mittel und
(c) ein mit einem ionischen organischen Tensid oder einem ionischen Makromolekül umgesetztes ionisches pharmazeutisches Mittel,
und einer zweiten Komponente, die ein wasserunlösliches Celluloseester-Polymeres enthält.

2. Zusammensetzung nach Anspruch 1, wobei das wasserunlösliche Polymere ferner mindestens einen der Bestandteile Polyurethanharze, Acryl-Polymere, Kondensationspolymere, Aldehyd-Kondensationspolymere und Polyisocyanate enthält.

3. Zusammensetzung nach Anspruch 1 oder 2, wobei es sich beim wasserunlöslichen Celluloseester-Polymeren um mindestens einen der Bestandteile Celluloseacetat, Celluloseacetobutyrat, Celluloseacetopropionat, Cellulosenitrat und Gemische davon handelt.

4. Zusammensetzung nach einem der vorstehenden Ansprüche, wobei die erste Komponente den Bestandteil (a) umfaßt und die Zusammensetzung antithrombogene Eigenschaften aufweist, wobei es sich bei der quaternären Ammoniumverbindung um mindestens einen der Bestandteile Benzalkoniumchlorid, Tridodecylmethylammoniumchlorid, Cetylpyridiniumchlorid, Benzyldimethylstearylammoniumchlorid und Benzylcetyldimethylammoniumchlorid handelt.

5. Zusammensetzung nach Anspruch 4, wobei es sich bei der quaternären Ammoniumverbindung um Benzalkoniumchlorid handelt.

6. Zusammensetzung nach Anspruch 4 oder 5, wobei (a) in einer Konzentration von mindestens 0,5 Gew.-% der Zusammensetzung vorhanden ist.

7. Zusammensetzung nach Anspruch 6, wobei (a) in einer Konzentration von 0,5 bis 99,5 Gew.-% der Zusammensetzung vorhanden ist, wobei es sich beim Rest um das wasserunlösliche Polymere handelt.

8. Zusammensetzung nach einem der Ansprüche 4 bis 6, wobei die erste Komponente ferner den Bestandteil (b) enthält, wobei das ionische organische Tensid eine quaternäre Ammoniumverbindung umfaßt.

9. Zusammensetzung nach einem der Ansprüche 1 bis 3, wobei die erste Komponente den Bestandteil (b) umfaßt und die Zusammensetzung antimikrobielle Eigenschaften aufweist, wobei das antibiotische Mittel negativ geladen ist und einen oder mehrere der Bestandteile Penicillin, Ticarcillin, Cefotoxin, Cephalosporine, Oxacillin und Carbonicillin enthält, wobei es sich beim Tensid um eine quaternäre Ammoniumverbindung handelt.

10. Zusammensetzung nach Anspruch 9, wobei der Bestandteil (b) in einer Konzentration von 0,5 bis 99,5 Gew.-% der Zusammensetzung vorhanden ist, wobei es sich beim Rest um das wasserlösliche Polymere handelt.

11. Zusammensetzung nach einem der Ansprüche 8 bis 10, wobei es sich bei der quaternären Ammoniumverbindung um mindestens einen der Bestandteile Benzalkoniumchlorid, Tridodecylmethylammoniumchlorid, Cetylpyridiniumchlorid, Benzyldimethylstearylammoniumchlorid und Benzylcetyldimethylammoniumchlorid handelt.

12. Zusammensetzung nach einem der Ansprüche 1 bis 3, wobei die erste Komponente den Bestandteil (b) umfaßt und die Zusammensetzung antimikrobielle Eigenschaften aufweist, wobei das antibiotische Mittel positiv geladen ist und das Tensid ein negativ geladenes organisches Ion aufweist.

13. Zusammensetzung nach Anspruch 10, wobei das Tensid Natriumlaurylsulfat oder Stearinsäure oder ein Salz davon oder Polyacrylsäure oder ein Copolymeres davon umfaßt.

14. Zusammensetzung nach einem der Ansprüche 8 bis 13, wobei der Bestandteil (b) und das wasserunlösliche Polymere jeweils in einer Konzentration von 0,01 bis 40 Gew.-% der Zusammensetzung vorhanden sind.

15. Zusammensetzung nach einem der vorstehenden Ansprüche, wobei sich die erste und die zweite Komponente jeweils in getrennten Schichten befinden.

16. Zusammensetzung, umfassend ein Gemisch nach einem der Ansprüche 1 bis 14 und ein wechselseitiges organisches Lösungsmittel, wobei die Konzentrationen der ersten und der zweiten Komponente im Lösungsmittel 0,1 bis 20 Gew.-% bzw. 0,01 bis 20 Gew.-% betragen.

17. Verfahren zur Bildung eines Überzugs auf der Oberfläche einer medizinischen Vorrichtung, die in Kontakt mit menschlichen oder tierischen Körperflüssigkeiten gebracht werden soll, wobei das Verfahren folgendes umfaßt:
(a) Herstellen einer Beschichtungslösung, die eine Zusammensetzung nach einem der Ansprüche 1 bis 14 und ein Co-Lösungsmittel für die erste und die zweite Komponente der Zusammensetzung umfaßt,
(b) Aufbringen der Beschichtungslösung auf die Oberfläche der Vorrichtung und
(c) Trocknenlassen der Lösung.

18. Verfahren zur Bildung eines Überzugs auf der Oberfläche einer medizinischen Vorrichtung, die in Kontakt mit menschlichen oder tierischen Körperflüssigkeiten gebracht werden soll, wobei das Verfahren folgendes umfaßt:
(a) Bilden von entsprechenden Beschichtungslösungen, die die erste und die zweite Komponente einer Zusammensetzung nach einem der Ansprüche 1 bis 14 und entsprechende Lösungsmittel umfassen,
(b) getrenntes Aufbringen der Beschichtungslösungen auf die Oberfläche der Vorrichtungen und
(c) Trocknenlassen der Lösungen.

19. Verfahren zur Bildung eines Überzugs auf der Oberfläche eines wasserunlöslichen Polymeren, wobei das Verfahren folgendes umfaßt:
(a) Bilden einer Beschichtungslösung, die die erste Komponente einer Zusammensetzung nach einem der Ansprüche 1 bis 14 und ein Lösungsmittel für die erste Komponente umfaßt,
(b) Aufbringen der Beschichtungslösung auf eine Oberfläche eines wasserunlöslichen Polymeren, das die zweite Komponente einer Zusammensetzung nach einem der Ansprüche 1 bis 14 umfaßt, und
(c) Trocknenlassen der Lösung.

20. Zusammensetzung nach einem der Ansprüche 17 bis 19, wobei die Konzentration der ersten Komponente in der jeweiligen Beschichtungslösung 0,1 bis 20 Gew.-% der Lösung beträgt.

21. Verfahren nach Anspruch 17 oder 18, wobei die Konzentration der zweiten Komponente in der jeweiligen Beschichtungslösung 0,01 bis 20 Gew.-% der Lösung beträgt.

22. Medizinische Vorrichtung, bei der mindestens ein Bereich ihrer Oberfläche mit der ersten Komponente einer Zusammensetzung nach einem der Ansprüche 1 bis 14 beschichtet ist, in gebundener Form mit der zweiten Komponente einer Zusammensetzung nach einem der Ansprüche 1 bis 14.

23. Medizinische Vorrichtung nach Anspruch 22, wobei es sich beim beschichteten Bereich um mindestens einen der Bestandteile Glas, Metall und Kunststoff handelt.

24. Medizinische Vorrichtung nach Anspruch 22 oder 23, wobei es sich bei der Vorrichtung um einen Katheter oder um ein künstliches Blutgefäß, ein künstliches Herz oder eine künstliche Niere handelt.

## Revendications

1. Composition comprenant un mélange d'un premier composant qui comprend l'une au moins des substances suivantes:
a) de l'héparine ayant reagi avec un compose d'ammonium quaternaire,
b) un agent antibiotique ionique ayant reagi avec un tensio-actif organique ionique ou une macromolécule ionique et
c) un agent pharmaceutique ionique ayant réagi avec un tensio-actif organique ionique ou une macromolécule ionique,
et d'un second composant comprenant un polymère d'ester de cellulose insoluble dans l'eau,

2. Composition selon la revendication 1, dans laquelle le polymère insoluble dans l'eau comprend en outre l'une au moins des substances suivantes; une résine de polyuréthanne, un polymère acrylique, un polymère de condensation, un polymère de condensation d'aldéhyde et un polyisocyanate.

3. Composition selon la revendication 1 ou 2, dans laquelle le polymère d'ester de cellulose insoluble dans l'eau est l'une au moins des substances suivantes; acétate de cellulose, acétate-butyrate de cellulose, acétate-propionate de cellulose, nitrate de cellulose et mélanges de ceux-ci.

4. Composition selon l'une quelconque des revendications précédentes, dans laquelle le premier composant comprend (a) et la composition a des propriétés antithrombose, le composé d'ammonium quaternaire étant l'une au moins des substances suivantes: chlorure de benzalkonium, chlorure de tridodécylméthylammonium, chlorure de cétylpyridinium, chlorure de benzyldiméthylstéarylammonium et chlorure de benzylcétyldiméthylammonium.

5. Composition selon la revendication 4, dans laquelle le composé d'ammonium quaternaire est le chlorure de benzalkonium.

6. Composition selon la revendication 4 au 5, dans laquelle (a) est présent à une concentration d'au moins 0,5% en poids de la composition.

7. Composition selon la revendication 6, dans laquelle (a) est présent à une concentration de 0,5% à 99,5% en poids de la composition, le reste étant constitué par le polymère insoluble dans l'eau.

8. Composition selon l'une quelconque des revendications 4 à 6, dans laquelle le premier composant contient aussi (b) dans lequel le tensio-actif organique ionique comprend un composé d'ammonium quaternaire.

9. Composition selon l'une quelconque des revendications 1 à 3, dans laquelle le premier composant comprend (b) et la composition a des propriétés antimicrobiennes, l'agent antibiotique étant chargé négativement et comprenant une ou plusieurs des substances pénicilline, ticaroilline, céfétoxine, céphalosporine, oxacilline et carbonicilline, le tensio-actif étant un composé d'ammonium quaternaire.

10. Composition selon la revendication 9, dans laquelle (b) est présent à une concentration de 0,5% à 99,5% en poids de la composition, le reste étant constitué par le polymère insoluble dans l'eau.

11. Composition selon l'une quelconque des revendications 8 à 10, dans laquelle le composé d'ammonium quaternaire est l'une au moins des substances suivantes; chlorure de benzalkonium, chlorure de tridodécylméthylammonium, chlorure de cétylpyridinium, chlorure de benzyldiméthylstéarylammonium et chlorure de benzylcétyldiméthylammonium.

12. Composition selon l'une quelconque des revendications 1 à 3, dans laquelle le premier composant comprend (b) et la composition a des propriétés antimicrobiennes, l'agent antibiotique étant chargé positivement et le tensio-actif comportant un ion organique chargé négativement.

13. Composition selon la revendication 10, dans laquelle le tensio-actif comprend le laurylsulfate de sodium, l'acide stéarique ou un sel de celui-ci, l'acide polyacrylique ou un copolymère de celui-ci.

14. Composition selon l'une quelconque des revendications 8 à 13, dans laquelle (b) et le polymère insoluble dans l'eau sont présents chacun à une concentration de 0,01% à 40% en poids de la composition.

15. Composition selon l'une quelconque des revendications 1 à 14, dans laquelle le premier et le second composants sont en phases séparées.

16. Composition comprenant un mélange selon l'une quelconque des revendications 1 à 14 et un solvant organique commun, les concentrations des premier et second composants dans le solvant étant respectivement de 0,1 à 20% en poids et de 0,01 à 20% en poids.

17. Procédé de formation d'un revêtement sur la surface d'un appareil médical qui doit être mis en contact avec des fluides corporels humains ou animaux, le procédé comprenant les opérations consistant:
a) à préparer une solution de revêtement qui comprend une composition selon l'une quelconque des revendications 1 à 14 et un co-solvant pour les premier et second composants de la composition,
b) à appliquer la solution de revêtement sur la surface de l'appareil et
c) à laisser la solution sécher.

18. Procédé de formation d'un revêtement sur la surface d'un appareil médical qui doit être mis en contact avec des fluides corporels humains ou animaux, le procédé comprenant les opérations consistant;
a) à préparer des solutions de revêtement qui comprennent respectivement le premier composant et le second composant d'une composition selon l'une quelconque des revendications 1 à 14 et des solvants respectifs,
b) à appliquer séparément les solutions de revêtement sur la surface de l'appareil et
c) à laisser les solutions sécher.

19. Procédé de formation d'un revêtement sur la surface d'un polymère insoluble dans l'eau, le procédé comprenant les opérations consistant:
a) à préparer une solution de revêtement qui comprend le premier composant d'une composition selon l'une quelconque des revendications 1 à 14 et un solvant pour le premier composant,
b) à appliquer la solution de revêtement sur une surface d'un polymère insoluble dans l'eau comprenant le second composant d'une composition selon l'une quelconque des revendications 1 à 14 et
c) à laisser la solution sécher.

20. Procédé selon l'une quelconque des revendications 17 à 19, dans lequel la concentration du premier composant dans sa solution de revêtement est de 0,1% à 20% en poids de la solution.

21. Procédé selon la revendication 17 ou 18, dans lequel la concentration du second composant dans sa solution de revêtement est de 0,01% à 20% en poids de la solution.

22. Appareil médical dont une partie au moins de la surface est revêtue du premier composant d'une composition selon l'une quelconque des revendications 1 à 14, lié au second composant d'une composition selon l'une quelconque des revendications 1 à 14.

23. Appareil médical selon la revendication 22, dans lequel la partie revêtue est en l'une au moins des matières verre, métal et plastique.

24. Appareil médical selon la revendication 22 ou 23, cet appareil étant un cathéter ou un vaisseau sanguin, coeur ou rein artificiel.
